# EUROPEAN PATENT APPLICATION

(11) **EP 1 752 437 A1**
(43) Date of publication of application: **14.02.2007**
(21) Application number: 04799641.8
(22) Date of filing: 05.11.2004
(51) Int. Cl.: C07C 45/33, C07C 45/35, C07C 47/22, C07C 51/215, C07C 51/225, C07C 51/235, C07C 57/05, C07C 57/055

(54) **PROCESS FOR PRODUCING (METH)ACRYLIC ACID OR (METH)ACROLEIN**

(30) Priority: 26.05.2004 JP 2004155840
(71) Applicant: MITSUBISHI CHEMICAL CORPORATION, Tokyo 108-0014 (JP)
(72) Inventor: OGAWA, Yasushi, Mitsubishi Chemical Corporation, Yokkaichi-shi Mie 5108530 (JP); YADA, Shuhei, Mitsubishi Chemical Corporation, Yokkaichi-shi Mie 5108530 (JP); SUZUKI, Yoshiro, Mitsubishi Chemical Corporation, Yokkaichi-shi Mie 5108530 (JP); TAKASAKI, Kenji, Mitsubishi Chemical Corporation, Yokkaichi-shi Mie 5108530 (JP); JINNO, Kimikatsu, Mitsubishi Chemical Corporation, Yokkaichi-shi Mie 5108530 (JP)
(74) Representative: Vossius & Partner
(86) International application number: PCT/JP2004/016789
(87) International publication number: WO 2005/115951

(57) **Abstract**

An object of the present invention is to provide a method for producing (meth)acrylic acid or (meth)acrolein by conducting a gas phase catalytic oxidation reaction with an oxygen-containing gas using as a raw material at least one substance to be oxidized selected from propylene, propane, isobutylene and (meth)acrolein using a multi-tubular reactor, which enables a high yield and stable production even when operating constantly with supplying the raw material in the maximum supply amount acceptable by the reactor or an amount close thereto.

The invention is a method for producing (meth)acrylic acid or (meth)acrolein wherein, at the time of a start-up of the reaction, for a period of at least 20 hours or more after the supply amount of the raw material to the reactor per unit time reached 30% or more of the acceptable maximum supply amount of the raw material per unit time, the supply amount of the raw material per unit time is kept at 30% or more and less than 80% of the acceptable maximum supply amount.

## Description

### <Technical Field>

The present invention relates to a method for producing (meth)acrylic acid or (meth) acrolein by a gas phase catalytic oxidation of at least one substance to be oxidized selected from propylene, propane, isobutylene and (meth)acrolein with a molecular oxygen using a multi-tubular reactor stably and efficiently.

### <Background Art>

(Meth)acrylic acid or (meth)acrolein is produced by a gas phase catalytic oxidation reaction in which propylene, propane, isobutylene or (meth)acrolein is brought into contact with a molecular oxygen or a molecular oxygen-containing gas in the presence of a composite oxide catalyst. This gas phase catalytic oxidation reaction is conducted usually with a multi-tubular reactor.

In such a reaction system, it is a matter of course that it is desirable to obtain an intended material stably at a high yield.

Based on the findings obtained newly by inventors of this invention, the (meth) acrylic acid or (meth)acrolein can be obtained stably at a high yield by using a certain contrivance at the time of a start-up of the reaction described above.

As a contrivance at the time of a start-up in a reaction system employing a catalytic gas phase oxidation reactor, a start-up method which is safe and can recycle its exhausted gas is proposed in Patent Reference 1 (JP-A-2001-53519). Patent Reference 2 (JP-A-2003-265948) also proposes a method for effecting a start-up efficiently without any adverse effect on the activity of a catalyst in a shell-tube type reactor which circulates a heat medium which is solid at ambient temperature.

### <Disclosure of the Invention>

An object of the present invention is to provide a method for producing (meth)acrylic acid or (meth)acrolein by conducting a gas phase catalytic oxidation reaction with an oxygen-containing gas using as a raw material at least one substance to be oxidized selected from propylene, propane, isobutylene and (meth)acrolein using a multi-tubular reactor, which enables a high yield and stable production even when operating constantly with supplying the raw material in the maximum supply amount acceptable by the reactor or an amount close thereto.

The inventors of this invention discovered that (meth)acrylic acid or (meth)acrolein can be produced more stably at a higher yield by employing a procedure in which, at the time of a start-up of the reaction, for a period of at least 20 hours or more after the supply amount of the raw material per unit time (hereinafter sometimes simply referred to as "supply amount") reached 30% or more of the acceptable maximum supply amount of the raw material to the reactor undergoing a stationary operation, the supply amount of the raw material is kept at the amount less than 80% of the acceptable maximum supply amount, and have achieved this invention based on this finding.

Thus, this invention is a method for producing (meth)acrylic acid or (meth)acrolein by conducting a gas phase catalytic oxidation reaction with an oxygen-containing gas using as a raw material at least one substance to be oxidized selected from propylene, propane, isobutylene and (meth)acrolein using a multi-tubular reactor, wherein, at the time of a start-up of the reaction, for a period of at least 20 hours or more after the supply amount of the raw material to the reactor per unit time reached 30% or more of the acceptable maximum supply amount of the raw material per unit time, the supply amount of the raw material per unit time is kept at 30% or more and less than 80% of the acceptable maximum supply amount.

In a prior method for producing acrolein for example from propylene, about 20 hours is required, at the time of the start-up, for raising the propylene supply amount from 30% to 100% of the acceptable maximum supply amount to the reactor.

Based on the inventors' researches, a conventional start-up allows some reaction tube to exhibit an abnormally elevated temperature, followed by a reduction in temperature (a rapid increase in the peak temperature followed by the loss of the temperature peak is observed by using a thermocouple). Such findings mean that a part which exhibits a specifically high activity (hereinafter referred to as an activity specific point) is present in the layer of a catalyst packed in a reaction tube and this activity specific point exhibits a high reactivity at the time of a start-up to cause a rapid increase in the temperature by which a surrounding catalyst is affected to result in a deactivation of the catalyst in the entire reaction tube (a loss of the temperature peak). Actually, when initiating the reaction by a conventional start-up and conducting a stationary operation with the acceptable maximum supply amount or a supply amount close thereto, the reaction yield was reduced by about 4% and the differential pressure between the inlet and the outlet of a deactivated reaction tube was higher by 3 times or more than that of a normal reaction tube. Such a deactivated reaction tube still remained at a frequency of about 5% after the stationary operation for one year.

On the contrary, when a time period during which the propylene supply amount is kept at 30% or more and less than 80%, for example kept at 70% of the acceptable maximum supply amount after reaching 30% of the acceptable maximum supply amount was 20 hours or more, for example, 10 days, according to a method of the invention, there was no reaction tube exhibiting an abnormally elevated temperature as described above. A subsequent stationary operation with the acceptable maximum supply amount or a supply amount close thereto for one year resulted in the differential pressure between the inlet and the outlet of a reaction tube which was same to that at the time of the initiation of the operation, with the catalytic activity being stable without undergoing any deactivation. The reaction yield was improved to about 2% when compared with a prior art. This may be attributable to a sufficient time period provided until a stationary operation near the acceptable maximum supply amount, which allows an activity specific point to disappear without affecting a surrounding catalyst.

### <Brief Description of the Drawings>

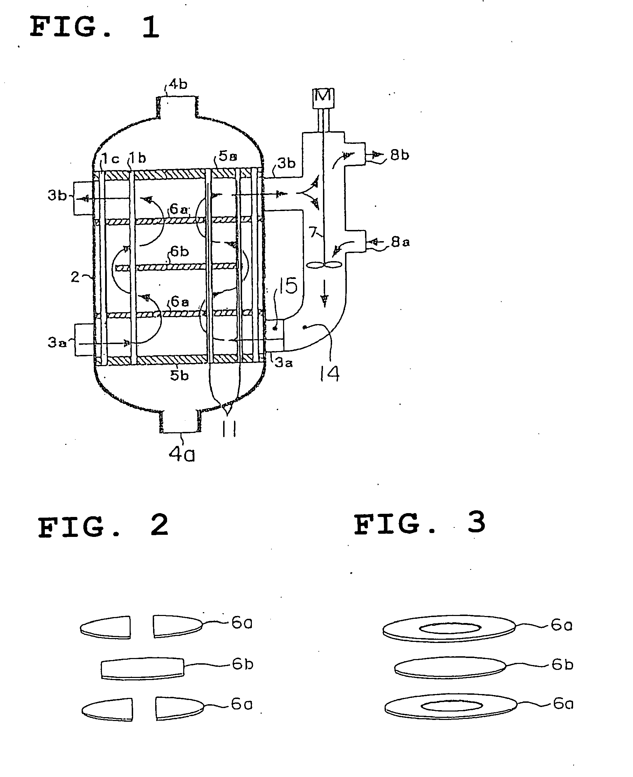

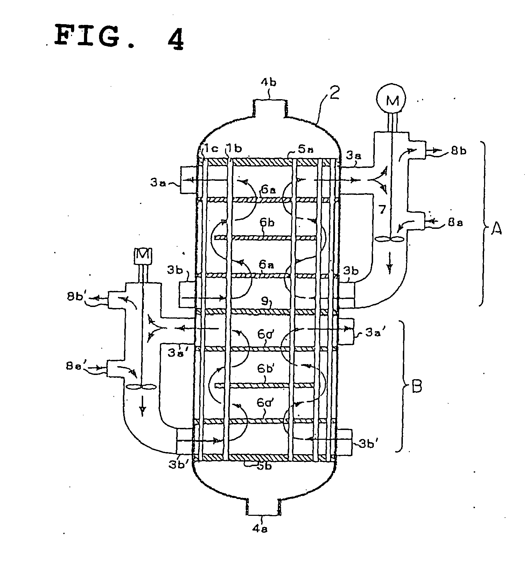

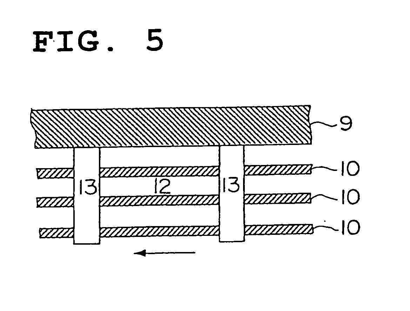

Fig. 1 shows a schematic sectional view indicating one embodiment of a multi-tubular heat exchange reactor employed in a gas phase catalytic oxidation method of the invention, Fig. 2 shows a schematic view indicating one embodiment of a baffle employed in a multi-tubular heat exchange reactor according to the invention, Fig. 3 shows a schematic view of another embodiment of the baffle which is different from that in Fig. 2, Fig. 4 shows a schematic sectional view of another embodiment of a multi-tubular heat exchange reactor employed in a gas phase catalytic oxidation method of the invention which is different from that in Fig. 1, Fig. 5 shows a magnified schematic sectional view of an intermediate tube plate which divides a shell in the multi-tubular heat exchange reactor shown in Fig. 4.

In this connection, the reference numerals 1b and 1c in the drawings are reaction tubes, 2 is a reactor, 3a and 3b are circular conduits, 3a' and 3b' are circular conduits, 4a is a product outlet, 4b is a raw material supply inlet, 5a and 5b are tube plates, 6a and 6b are holed baffles, 6a' and 6b' are holed baffles, 7 is a circulation pump, 8a and 8a' are heat medium supply lines, 8b and 8b' are heat medium draining line, 9 is an intermediate tube plate, 10 is a heat shielding plate, 11, 14 and 15 are thermometers, 12 is a stagnation space, and 13 is a spacer rod.

### <Best Mode for Carrying Out the Invention>

The invention is detailed below.

The method of the invention is a method for producing (meth)acrylic acid or (meth)acrolein by conducting a gas phase catalytic oxidation reaction using a multi-tubular reactor packed with a catalyst effecting a gas phase catalytic oxidation using as a raw material at least one substance to be oxidized selected from propylene, propane, isobutylene and (meth)acrolein, characterized in that a sufficient time period is provided until a stationary operation near the acceptable maximum supply amount by adjusting the raw material supply amount at the time of a start-up of the reaction.

As used herein, the term "acceptable maximum supply amount" means a maximum amount of a raw material allowed to be supplied to a reactor per unit time. This value correlates with the production capability of the reactor and determined at the stage of the designing of the reactor.

In the invention, at the time of a start-up of the reaction, for a period of at least 20 hours or more, preferably 24 hours or more and not more than 80 hours, after the supply amount reached 30% or more of the acceptable maximum supply amount, the supply amount of the raw material to the reactor is kept at 30% or more and less than 80%, preferably 50% or more and not more than 75%, as mentioned above. As a result, the effects of the invention can be exerted and the (meth)acrylic acid or the (meth)acrolein can be produced stably at an improved yield even when operating with the acceptable maximum supply amount.

The reaction systems, reactors, catalysts and the like employed in the invention are described below.

### (Reaction systems)

While a representative of the reaction systems in an industrial method for producing an acrolein and acrylic acid may for example be a one-path system, an unreacted propylene recycling system and a combustion exhaust gas recycling system, the invention is not limited to any reaction systems including these three systems.

### (1) One-path system:

In this system, propylene, air and steam are supplied as being mixed at a former stage reaction to convert mainly into an acrolein and acrylic acid, the outlet gas of which is supplied to a latter stage reaction (mainly converting the acrolein to the acrylic acid) without separating from the products. In this procedure, it is common to supply air and steam required in the reaction at the latter stage in addition to the outlet gas from the former stage, to the reaction at the latter stage.

### (2) Unreacted propylene recycling system:

In this system, the reaction product gas containing the acrylic acid obtained in the latter stage reaction is introduced into acrylic acid trapping device, where the acrylic acid is trapped as an aqueous solution, and a part of the exhausted gas containing unreacted propylene in this acrylic acid trapping device is supplied to the former stage reaction, whereby recycling a part of the unreacted propylene.

### (3) Combustion exhaust gas recycling system:

In this system, the reaction product gas containing the acrylic acid obtained in the latter stage reaction is introduced into acrylic acid trapping device, where the acrylic acid is trapped as an aqueous solution, all of the exhausted gas in this acrylic acid trapping device is combusted and oxidized to convert the contained unreacted propylene and the like mainly into carbon dioxide and water, and a part of the combusted exhausted gas thus obtained is added to the former stage reaction.

Generally, a multi-tubular reactor is used for the purpose of increasing the productivity of the reactor while protecting a catalyst and keeping the performance of the catalyst at a high level by controlling the catalyst reaction temperature precisely due to an extremely large exothermic heat as in an oxidation reaction.

Recently, the amount of the production of acrylic acid from propylene and methacrylic acid from isobutylene (together referred to as (meth)acrylic acid) was increased greatly in response to an increased demand, and a large number of plants were built in the world, with the plant production scale being increased to 100 thousand tons or more per plant per year. As a result of an increased plant production scale, the amount produced by a single oxidation reactor should be increased, resulting in an increased load of a gas phase catalytic oxidation reactor of propane, propylene or isobutylene. Accordingly, a multi-tubular reactor is needed to be imparted with a far higher performance.

In the invention, a method for a gas phase catalytic oxidation of a substance to be oxidized with a molecular oxygen-containing gas using a multi-tubular reactor having, in its longitudinal direction of the reaction tubes, a cylindrical reactor shell having a raw material supply inlet and a product outlet, a plural of circular conduits provided on the outer circumference of the cylindrical reactor shell for allowing a heat medium to come into or go out of the cylindrical reactor shell, a circulating device connecting the plurality of the circular conduits with each other, a plural of reaction tubes which are restrained by a plural of the tube plate of the reactor and which contain catalysts and a plural of baffles for changing the direction of the heat medium allowed to come into the reactor shell is employed, and the reaction tube described above is packed with an oxidation catalyst such as an Mo-Bi-based catalyst and/or an Mo-V-based catalyst.

The invention is a gas phase catalytic oxidation method which employs propylene, propane, isobutylene or (meth)acrolein or a mixture thereof as a substance to be oxidized and a gas phase catalytic oxidation is conducted using a molecular oxygen-containing gas to obtain (meth)acrolein or (meth)acrylic acid. (Meth)acrolein, (meth)acrylic acid or both are obtained from propylene, propane, isobutylene. (Meth)acrylic acid is obtained also from (meth)acrolein.

As used herein, a "process gas" means a gas involved in a gas phase catalytic oxidation reaction including a substance to be oxidized and a molecular oxygen-containing gas as raw material gases, resultant products and the like. A "raw material" means a substance to be oxidized.

### (Raw material gas composition)

To a multi-tubular reactor employed in a gas phase catalytic oxidation, a gas mixture of at least one substance to be oxidized selected from propylene, propane, isobutylene and (meth)acrolein, a molecular oxygen-containing gas and water vapor is mainly introduced as a raw material gas.

In the invention, the concentration of the substance to be oxidized in the raw material gas is 6 to 10% by mole, with the oxygen being in an amount of 1.5 to 2.5 molar times and the water vapor in an amount of 0.8 to 5 molar times that the substance to be oxidized. The raw material gas introduced passes through the reaction tubes as being divided into each reaction tube, and reacts in the presence of the packed oxidizing catalyst.

### (Multi-tubular reactor)

A gas phase catalytic oxidation reaction according to the invention which employs a multi-tubular reactor is a method employed widely for producing (meth)acrylic acid or (meth)acrolein using a molecular oxygen or a molecular oxygen-containing gas in the presence of a composite oxide catalyst from at least one substance to be oxidized selected from propylene, propane, isobutylene and (meth)acrolein.

A multi-tubular reactor employed in the invention is not limited particularly and may be one employed industrially.

One embodiment of a multi-tubular reactor employed in the invention is described with referring to Figs. 1 to 5.

### (Fig. 1)

Fig. 1 shows a schematic sectional view indicating one embodiment of a multi-tubular heat exchange reactor employed in a gas phase catalytic oxidation method of the invention.

In a shell 2 of the multi-tubular reactor, reaction tubes 1b and 1c are fixed on tube plates 5a and 5b. A raw material supply inlet which is an inlet of raw material gas for the reaction and a product outlet which is an outlet of a product are 4a or 4b. While the direction of the flow of a process gas may be in any way when the flows of a process gas and a heat medium are countercurrents, 4b is the raw material supply inlet in Fig. 1 since the direction of the flow of the heat medium in the reactor shell is indicated upward by an arrow. On the outer circumference of the reactor shell, circular conduits 3a for allowing the heat medium to come in are provided. The heat medium whose pressure has been raised by a circulation pump 7 for the heat medium goes from the circular conduits 3a up through the reactor shell with its direction of the flow being converted due to a plural of alternatively located holed baffles 6a having openings near the center of the reactor shell and holed baffles 6b placed to form openings between the circumference of the reactor shell, whereby returning to the circulation pump through circular conduits 3b. A part of the heat medium absorbing the reaction heat is passed through a discharge pipe provided on the top of the circulation pump 7 with being cooled by a heat exchanger (not shown) to go into a heat medium supplying line 8a to be re-introduced into the reactor. The adjustment of the heat medium temperature is accomplished by adjusting the temperature or the flow rate of the refluxing heat medium introduced from the heat medium supplying line 8a to control the temperature based on the temperature detected by a thermometer 14.

The temperature of the heat medium is adjusted so that the difference in the temperature between the heat medium supplying line 8a and the heat medium draining line 8b is 1 to 10°C, preferably 2 to 6°C, although it depends on the performance of the catalyst employed.

It is preferred to provide a current plate (not shown) on the body plate part inside of the circular conduits 3a and 3b for the purpose of minimizing the distribution of the heat medium flow speed toward the direction of the circumference. As the current plate, a porous plate or a slit plate is employed, and the opening area of the porous plate or the slit gap may be changed to achieve a rectification effect which allows the heat medium to come in at a similar flow speed from all over the circumference. The temperature inside the circular conduits (3a, preferably also 3b) can be monitored by providing a single or a plural of thermometers 15.

While the number of the baffles provided in the reactor shell is not limited particularly, it is preferable to provide three plates (2 plates of 6a type and 1 plate of 6b type) as in an ordinary case. By the existence of these baffles, the upward flow of the heat medium is prevented, and converted into a lateral direction with respect to the axial direction of the reactor tube, whereby allowing the heat medium to be collected from the circumference to the center of the reactor shell, and then to be turned around toward the circumference at the opening of the baffle 6a, then allowed to reach the outer cylinder of the shell. The heat medium is turned around again at the circumference of the baffles 6b to be collected into the center, and then goes upward through the openings of the baffles 6a to go along the upper tube plate 5a of the reactor shell toward the circumference, and then passes through the circular conduits 3b to circulate to the pump.

Into a plural of the reaction tubes provided in the reactor, the thermometers 11 are inserted, and the signals are transmitted to the outside of the reactor, and the temperature distribution in the catalyst layer in the direction of the reactor tube axis is recorded. In a plural of the reaction tubes having the thermometers inserted thereinto, a single thermometer can measure the temperature at 5 to 20 points in the direction of the tube axis.

### (Fig. 2, Fig. 3: Baffles)

A baffle employed in the invention may be any of a segment type chipped circular baffle shown in Fig. 2 or a disc type baffle shown in Fig. 3, provided that a baffle 6a has an opening near the center of the reactor shell while a baffle 6b forms an gap between its circumference and the outer cylinder of the shell and the heat medium is turned around at each opening to prevent any by-passing of the heat medium and to change the flow speed. In both types of the baffles, the relationship between the heat medium flow direction and the reaction tube axis is not changed.

As an ordinary baffle, a disc baffle shown in Fig. 3 is employed widely. The area of the central opening of the baffle 6a is preferably 5 to 50%, more preferably 10 to 30% of the sectional area of the reactor shell. The area of the gap between the baffle 6b and the reactor shell body plate 2 is preferably 5 to 50%, more preferably 10 to 30% of the sectional area of the reactor shell. A too low opening ratio of the baffles (6a and 6b) leads to a too long flow path of the heat medium which results in an increased pressure loss between the circular conduits (3a and 3b) which causes an increased power of the heat medium circulation pump 7. A too high opening ratio of the baffles leads to an increased number of the reaction tubes (1c).

While the distance between the respective baffles (the distance between the baffles 6a and 6b as well as the distance between the baffle 6a and the tube plates 5a, 5b) is frequently equal, it is not necessarily equal. It may be adjusted appropriately so that the required flow rate of the heat medium determined on the basis of the oxidation reaction heat generated in the reaction tubes is surely obtained and the pressure loss of the heat medium is low.

### (Fig. 4)

Fig. 4 shows a schematic sectional view of a multi-tubular reactor in which the reactor shell is divided by an intermediate tube plate 9, and the gas phase catalytic oxidation method of the invention also encompasses a method using a reactor of this type. In each space formed by the division, a discrete heat medium is circulated, and the temperature is controlled discretely. While a raw material gas may be introduced via either of 4a or 4b, 4b is the raw material supply inlet in Fig. 4 which makes the flow of the raw material process gas a countercurrent with respect to the heat medium flow since the direction of the flow of the heat medium in the reactor shell is indicated upward by an arrow. The raw material gas introduced via the raw material supply inlet 4b is reacted sequentially in the reaction tubes in the reactor.

Since the temperature of the heat medium is different between the upper and lower areas (area A and area B in Fig. 4) partitioned with the intermediate tube plate 9 in the multi-tubular reactor shown in Fig. 4, the inside of the reactor is in the following three cases: 1) a case in which an identical catalyst is packed entirely and the reaction is effected with different temperatures between the inlet and the outlet of the raw material gases of the reaction tubes, 2) a case in which the catalyst is packed at the inlet of the raw material gases but the outlet is not packed with a catalyst and allowed to be vacant or is rather packed with an inert substance having no reaction activity for the purpose of cooling a reaction product rapidly, and 3) a case in which different catalysts are packed at the inlet and the outlet of the raw material gases, the region between which is not packed with a catalyst and allowed to be vacant or is rather packed with an inert substance having no reaction activity for the purpose of cooling a reaction product rapidly.

For example, into the multi-tubular reactor employed in the invention shown in Fig. 4, a gas mixture of propylene, propane or isobutylene with a molecular oxygen-containing gas is introduced via the raw material supply inlet 4b, and firstly converted into (meth)acrolein in the first stage for a former stage reaction (area A in the reaction tubes) and then the (meth)acrolein is oxidized in the second stage for a latter stage reaction (area B in the reaction tubes) to produce (meth)acrylic acid. In this example, the first stage (hereinafter sometimes referred to as "former stage") and the second stage (hereinafter sometimes referred to as "latter stage") of the reaction tubes are packed with different catalysts, which are controlled at different temperature to effect the reaction under an optimum condition. It is preferred that the region where the intermediate tube plate is provided between the former stage and the latter stage of the reaction tubes is packed with an inert substance which is not involved in the reaction.

### (Fig. 5)

An intermediate plate is shown in Fig. 5 as being magnified. While the former stage and the latter stage are controlled at different temperature, a difference in the temperature exceeding 100°C causes a non-negligible heat transfer from a higher temperature heat medium to a lower temperature heat medium which may lead to a reduced accuracy of the reaction temperature of the lower temperature side. In such a case, a heat insulation for preventing the heat transfer between the upper and lower regions of the intermediate tube plate is required. In Fig. 5 showing the case employing the heat insulation plate, two or three heat insulation plates 10 are provided at a position of about 10 cm below or above the intermediate tube plate to form a stagnation zone 12 in which there is no flow but which is filled with the heat medium, whereby obtaining a preferable heat insulation effect. The heat insulation plates 10 are fixed on the intermediate tube plate 9 for example by spacer rods 13.

Although the direction of the flow of the heat medium in the reactor shell is indicated upward by an arrow in Fig. 1 and Fig. 4, the reverse direction may be used in the present invention. For a decision with regard to the direction of the circulation of the heat medium, a care must be taken to prevent any migration of a gas which may be present on the upper edge of the reactor shell 2 and the circulation pump 7, typically an inert gas such as nitrogen, into the heat medium flow. When the heat medium flows upward (Fig. 1), any migration of the gas at the upper region of the circulation pump 7 may result in a cavitation in the circulation pump, which may lead to a worst consequence such as a breakage of the pump. When the heat medium flows downward, the gas migration occurs at the top of the reactor shell to form a gas phase stagnation in the upper region of the shell which prevents the heat medium from cooling the upper region of the reaction tubes around such a gas stagnation.

To prevent the formation of such a gas stagnation, a gas exhausting line should be provided to replace the gas in the gas layer with the heat medium. For this purpose, the heat medium pressure in the heat medium supplying line 8a is increased when the heat medium flows upward (Fig. 1) and the heat medium draining line 8b is placed as high as possible, whereby increasing the pressure in the shell. The heat medium draining line is located preferably above the tube plate 5a.

In a multi-tubular reactor which oxidizes propylene, propane or isobutylene with a molecular oxygen-containing gas, when the multi-tubular reactor shown in Fig. 1 is employed and the process gases flow downward, i.e., the raw material gas is introduced from 4b and the product is put out of 4a, then the concentration of the intended product (meth)acrolein becomes high near the product outlet 4a of the reactor where heating by the reaction heat also raises the temperature of the process gases. Accordingly, in such a case, it is preferable to provide a heat exchange device following to 4a of the reactor shown in Fig. 1 to cool the process gases sufficiently whereby preventing any auto-oxidation of the (meth)acrolein.

Also when the multi-tubular reactor shown in Fig. 4 is employed and the process gases flow downward, i.e., the raw material gas is introduced from 4b and the product is put out of 4a, then the concentration of the intended product (meth)acrolein becomes high near the intermediate tube plate 9 at the endpoint of the reaction of the first stage (area A in the reaction tubes) where heating by the reaction heat also raises the temperature of the process gases. When the catalyst is packed only in the first stage (area A in the reaction tubes: 5a-6a-6b-6a-9), then the reaction is not conducted in the second stage of the reaction tubes 1b, 1c (area B in the reaction tube: between 9 to 5b) where the process gases are cooked by the heat medium flowing through the channel on the side of the shell whereby preventing any auto-oxidation of the (meth)acrolein. In such a case, it is preferable that the area B in the reaction tubes 1b, 1c (between 9 and 5b) is not packed with a catalyst and is allowed to be vacant or is rather packed with a solid having no reaction activity. The latter is preferable for the purpose of improving the heat transmission profile.

Also when the first stage of the multi-tubular reactor shown in Fig. 4 (area A in the reaction tubes: 5a-6a-6b-6a-9) and the second stage (area B in the reaction tube:9-6a'-6b'-6a'-5b) are packed with different catalysts to obtain (meth)acrolein from propylene, propane or isobutylene on the first stage and obtain (meth)acrylic acid on the second stage, the catalyst layer temperature on the first stage is higher than that the catalyst layer temperature on the second stage. Typically, the temperature becomes higher near the reaction endpoint of the first stage (6a-9) and the reaction starting point of the second stage (9-6a'), where it is preferred that no reaction is conducted and the process gases are cooled by the heat medium flowing through the channel on the side of the shell whereby preventing any auto-oxidation of the (meth)acrolein. In such a case, a zone is provided near the intermediate tube plate 9 (6a-9-6a' in reaction tubes 1b, 1c) which is not packed with a catalyst and is allowed to be vacant or is rather packed with a solid having no reaction activity. The latter is preferable for the purpose of improving the heat transmission profile.

### (Reaction tube diameter)

The inner diameter of a reaction tube having an effect on the gas line speed is extremely important, since the inside of the reaction tube containing an oxidation catalyst in an oxidation reactor is in a gas phase, and also since the gas line speed is limited due to a resistance by the catalyst and the heat transmission coefficient in the tube is the lowest and allows the heat transmission to be a rate determinant.

While the inner diameter of a reaction tube of a multi-tubular reactor according to the invention may vary depending on the reaction heat amount and the catalyst particle size in the reaction tube, it is preferably 10 to 50 mm, more preferably 20 to 30 mm. A too small inner diameter of the reaction tube leads to a reduced amount of the catalyst to be packed which leads to an increased number of the reaction tubes relative to the amount of the catalyst required, resulting in a requirement of a high production cost due to increased labor at the time of the reactor production which is disadvantageous in view of an industrial efficiency. On the other hand, a too large inner diameter of the reaction tube leads to a reduced surface area of the reaction tube relative to the amount of the catalyst required, resulting in a reduction in the heat transmission area for removing the reaction heat.

### (Catalyst)

As a catalyst employed in a gas phase catalytic oxidation for producing (meth)acrylic acid or (meth)acrolein, there is one for a first stage reaction converting an olefin to an unsaturated aldehyde or an unsaturated acid and one for a second stage reaction converting an unsaturated aldehyde to an unsaturated acid.

In the gas phase catalytic oxidation reaction described above, an Mo-Bi-based composite oxidation catalyst employed in the first stage reaction mainly for producing acrolein (reaction for converting an olefin to an unsaturated aldehyde or an unsaturated acid) may for example be one represented by Formula (I) shown below:

Formula (I) MoₐW_{b}Bi_{c}Fe_{d}AₑB_{f}C_{g}DₕEᵢOₓ

In Formula (I) shown above, A denotes at least one element selected from nickel and cobalt, B denotes at least one element selected from sodium, potassium, rubidium, cesium and thallium, C denotes at least one element selected from alkaline earth metals, D denotes at least one element selected from phosphorus, tellurium, antimony, tin, cerium, lead, niobium, manganese, arsenic, boron and zinc, E denotes at least one element selected from silicon, aluminum, titanium and zirconium, and O denotes oxygen. a, b, c, d, e, f, g, h, i and x denotes the atomic ratios of Mo, W, Bi, Fe, A, B, C, D, E and O, respectively, and when a is 12 then b is 0 to 10, c is 0 to 10 (preferably 0.1 to 10), d is 0 to 10 (preferably 0.1 to 10), e is 0 to 15, f is 0 to 10 (preferably 0.001 to 10), g is 0 to 10, h is 0 to 4, i is 0 to 30, x is a value determined depending-on the oxidation state of each element.

In the gas phase catalytic oxidation reaction described above, an Mo-V-based composite oxidation catalyst employed in the second stage reaction for oxidizing acrolein to produce acrylic acid (reaction for converting an unsaturated aldehyde to an unsaturated acid) may for example be one represented by Formula (II) shown below:

Formula (II) MOₐV_{b}W_{c}CU_{d}XₑY_{f}O_{g}

In Formula (II) shown above, X denotes at least one element selected from Mg, Ca, Sr and Ba, Y denotes at least one element selected from Ti, Zr, Ce, Cr, Mn, Fe, Co, Ni, Zn, Nb, Sn, Sb, Pb and Bi, and O denotes oxygen. a, b, c, d, e, f and g denotes the atomic ratios of Mo, V, W, Cu, X, Y and O, respectively, and when a is 12 then b is 2 to 14, c is 0 to 12, d is 0 to 6, e is 0 to 3, Of is 0 to 3, and g is a value determined depending on the oxidation state of each element.

A catalyst described above may be produced by a method described for example in JP-A-63-54942, JP-B-6-13096, JP-B-6-38918 and the like.

A catalyst employed in the invention may be a molded catalyst obtained by an extrusion molding or a tablet compression, or may be a supported catalyst formed by allowing a composite oxides consisting of catalyst components to be supported on an inert carrier such as silicon carbide, alumina, zirconium oxide, titanium oxide and the like.

The shape of a catalyst employed in the invention is not limited particularly, and may be any shape such as sphere, column, cylinder, star and ring or may be amorphous.

### (Diluent)

A catalyst described above may be used as a mixture with an inert substance as a diluent.

While such an inert substance is not limited particularly as long as it is stable under a reaction condition and is not reactive with a raw material substance or a product, it is preferably one employed as a carrier for a catalyst, such as alumina, silicon carbide, silica, zirconium oxide, titanium oxide and the like.

Its shape is not limited particularly similarly to a catalyst, and may be any shape such as sphere, column, cylinder, star, ring, chip and network or may be amorphous. The size may be determined while taking the reaction tube diameter and the pressure loss into consideration.

The amount of an inert substance as a diluent may be determined appropriately based on the intended catalytic activity.

### (Catalyst layer, activity control and the like)

The activity of a catalyst layer in a reaction tube can be changed.

A method for the adjustment for changing the activity of a catalyst layer in a reaction tube may for example be a way to adjust the composition of the catalysts to give catalysts having different activities to be used in respective catalyst layers, or a way to mix a catalyst particle with an inert substance particle to dilute the catalyst whereby adjusting the activity of each catalyst layer.

In a typical example of the latter way, the catalyst layers consists of two layers, namely a low activity layer which is a catalyst layer at the inlet of the raw material gases in a reaction tube where an inert substance particle is contained at a higher level and the amount of the inert substance particle (ratio by mass) may for example be 0.3 to 0.7 based on the catalyst, and a high activity layer which is a catalyst layer at the outlet of the reaction tube where such a ratio is as low as 0 to 0.5 or a non-diluted catalyst is packed.

While the number of the catalyst layers formed in the direction of the tube axis of a multi-tubular reactor is not limited, the number of the catalyst layers is usually 1 to 10 since a too large number of the catalyst layers leads to a requirement of an enormous labor for packing the catalyst. The optimum length of each catalyst layer may vary depending on the catalyst type, the number of the catalyst layers, the reaction conditions and the like, and may be determined appropriately for allowing a maximum effect of the invention to be exerted.

An auxiliary matter of the invention is discussed below.

### (Step for producing acrylic acid or acrylates)

A step for producing acrylic acid may for example be the steps (i) to (iii) shown below. In any step, the technique described above is taken.
(i) An oxidation step for effecting a catalytic gas phase oxidation of propane, propylene and/or acrolein, a collection step for bringing an acrylic acid-containing gas from the oxidation step into contact with water to collect the acrylic acid as an aqueous solution of acrylic acid, an extraction step for extracting the acrylic acid from this aqueous solution of the acrylic acid using a suitable extraction solvent, and subsequent separation of the acrylic acid from the solvent followed by a purification step are provided, and then a high boiling fluid containing a Michael adduct of the acrylic acid and polymerization inhibitors employed in respective steps is supplied as a raw material to a decomposition reaction tower to recover valuable materials (for example, acrylic acid) and the valuable materials are supplied to any step of the collection step or later steps.
(ii) An oxidation step for effecting a catalytic gas phase oxidation of propylene, propane and/or acrolein to produce acrylic acid, a collection step for bringing an acrylic acid-containing gas into contact with water to collect the acrylic acid as an aqueous solution of acrylic acid, an azeotropic separation step for distilling this aqueous solution of the acrylic acid in an azeotropic separation tower in the presence of an azeotropic solvent to collect a crude acrylic acid from the tower bottom, and then an acetic acid separation step for removing acetic acid, followed by a purification step for removing high boiling impurities are provided, and then a high boiling fluid containing a Michael adduct of the acrylic acid and polymerization inhibitors employed in these production steps is supplied as a raw material to a decomposition reaction tower to recover valuable materials (for example, acrylic acid) and the valuable materials are supplied to any step of the collection step or later steps.
(iii) An oxidation step for effecting a catalytic gas phase oxidation of propylene, propane and/or acrolein to produce acrylic acid, a collection/separation step for bringing an acrylic acid-containing gas into contact with an organic solvent to collect the acrylic acid as an organic solution of acrylic acid while removing water, acetic acid and the like simultaneously, a separation step for isolating the acrylic acid from this organic solution of the acrylic acid, a step in which a high boiling fluid containing polymerization inhibitors employed in these production steps, organic solvents and a Michael adduct of the acrylic acid is supplied as a raw material to a decomposition reaction tower to recover valuable materials and the valuable materials are supplied to any step of the collection step or later, and a step for purifying a part of the organic solvent are provided.

A step for producing an acrylate consists for example of an esterification reaction step for reacting acrylic acid with an alcohol using an organic acid or a cationic ion exchange resin as a catalyst and a purification step for conducting extraction, evaporation and distillation each as a unit operation for condensing the crude acrylate solution obtained in the reaction. Each unit operation may be selected appropriately based on the ratio of the acrylic acid and the alcohol as raw materials in the esterification reaction, the type of the catalyst employed for the esterification reaction, or the physical properties of the raw materials, reaction by-products and acrylates. Through respective unit operations, a product is obtained in an acrylate purification tower. The fluid on the bottom of the purification tower may be supplied to a decomposition reaction tower as a high boiling fluid containing a Michael adduct whose main components are acrylates, β-acryloxypropionates, β-alkoxypropionates, β-hydroxypropionates together with polymerization inhibitors employed in the production steps, or may be returned to the process whereby recovering the valuable materials.

In the production of acrylic acid or acrylates which are readily polymerizable compounds, a polymerization inhibitor is employed to suppress the formation of the polymers during the production.

Typically, such a polymerization inhibitor may for example be copper acrylate, copper dithiocarbamate, phenolic compounds, phenothiazine compounds and the like. The copper dithiocarbamate may for example be a copper dialkyldithiocarbamate such as copper dimethyldithiocarbamate, copper diethyldithiocarbamate, copper dipropyldithiocarbamate, copper dibutyldithiocarbamate and the like, a copper cycloalkylenedithiocarbamate such as copper ethylenedithiocarbamate, copper tetramethylene dithiocarbamate, copper pentamethylene dithiocarbamate, copper hexamethylenedithiocarbamate and the like, and a copper cyclooxydialkylenedithiocarbamate such as copper oxydiethylenedithiocarbamate and the like. The phenolic compound may for example be hydroquinone, methoquinone, pyrogallol, cathecol, resorcine, phenol, cresol and the like. The phenothiazine compound may for example be phenothiazine, bis-(α-methylbenzyl)phenothiazine, 3,7-dioctylphenothiazine, bis(α-dimethylbenzyl)phenothiazine and the like.

While substances other than those listed above may be involved in some processes, their types clearly have no effects on the invention.

Acrylic acid or acrylates thus obtained can be used in various applications. Typically, it may be used in a highly absorptive resin, coagulant, pressure-sensitive adhesive, paint, adhesive, fiber modifier and the like.

### <Examples>

The invention is further described in the following Example and Comparative Example which are not intended to restrict the invention.

### [Example 1]

### (Catalyst)

94 Parts by mass of antimony paramolybdate was dissolved in 400 parts by mass of pure water with heating. On the other hand, 7.2 parts by mass of ferric nitrate, 25 parts by mass of cobalt nitrate and 38 parts by mass of nickel nitrate were dissolved in 60 parts by mass of pure water with heating. These solutions were mixed with stirring thoroughly to obtain a slurry solution.

Then, 0.85 parts by mass of borax and 0.36 parts by mass of potassium nitrate were dissolved in 40 parts by mass of pure water with heating and then added to the slurry described above. Then 64 parts by mass of particulate silica was added and stirred. Then 58 parts by mass of bismuth subcarbonate which had previously be made composite with 0.8% by mass of Mg was added and mixed with stirring, and this slurry was died with heating, and then heat-treated for 1 hour at 300°C in air atmosphere, and the resultant particulate solid was subjected to tablet compression using a molding machine into tablets each being 5 mm in diameter and 4 mm in height, and then sintered for 4 hours at 500°C to obtain a former stage catalyst.

The former stage catalyst thus obtained was an Mo-Bi-based composite oxide having the composition ratio of a catalyst powder whose formula was Mo₁₂Bi₅Ni₃Co₂Fe_{0.4}Na_{0.2}Mg_{0.4}B_{0.2}K_{0.1}Si₂₄O₂₅ (the oxygen composition ratio x is a value determined depending on the oxidation state of each metal.

### (Production of acrylic acid and acrolein from propylene)

In this Example, a multi-tubular reactor similar to that shown in Fig. 1 was employed.

Typically, a multi-tubular reactor of a reaction shell (inner diameter: 4,500 mm) having 10,000 stainless steel-made reaction tube each being 3.5 m in length and 27 mm in inner diameter was employed. No reaction tube was provided in the round opening region in the center of a holed disc baffle 6a having an opening near the center of the reactor shell. The baffles consisted of holed disc baffles 6a each having an opening near the center of the reactor shell and a holed disc baffle 6b which formed a gap between the circumference of the reactor, which were located at equal intervals in the order of 6a-6b-6a, with the opening ratio of each baffle being 18%.

A catalyst to be packed in each reaction tube was obtained by mixing the former stage catalyst described above with silica-made balls each having no catalytic activity and being 5 mm in diameter to adjust the catalytic activity, and packed in such a manner that the ratio of the catalytic activities became 0.5, 0.7 and 1 from the inlet of the reaction tube, whereby forming three catalyst layers.

### (Start-up method)

A heat medium (NITER) which was an inorganic mixed salt was passed through the side of the reactor shell to keep the temperature at 330°C. Prior to the supply of propylene, 1845 Nm³/hr of oxygen, 8241 Nm³/hr of nitrogen and 1107 Nm³/hr of water vapor were supplied to the reactor and then the catalytic layer temperature was ensured to be almost similar to that of the NITER, and thereafter the supply of propylene was started.

The propylene supply was reached 340 Nm³/hr 2 hours after the start, and then the supply amount was increased by 50 Nm³/hr per hour to reach 775 Nm³/hr (corresponding to about 70% of the maximum supply amount) about 11 hours after the start. The NITER temperature was kept at 330°C for 12 hours.

Then the propylene supply amount was increased over about 70 minutes until 830 Nm³/hr (corresponding to 75% of the maximum supply amount). The NITER temperature was kept at 331°C for 24 hours.

Then the propylene supply amount was increased over about 200 minutes until 996 Nm³/hr (corresponding to 90% of the maximum supply amount), and the NITER temperature was kept at 333°C for 4 hours, followed by an elevation to 1107 Nm³/hr (corresponding to 100% of the maximum supply amount) over about 130 minutes, and then the NITER temperature was set at 335°C for switching into a stationary operation.

At this time, the raw material gas composition consisted of 9% by mole of propylene, 15% by mole of oxygen, 9% by mole of water vapor, 67% by mole of nitrogen, with the pressure being 75 kPa (gauge pressure) and the gas supply amount being 12300 Nm³/hr.

### (Stationary operation)

When operating for a prolonged period, the NITER temperature was adjusted so that the % propylene conversion became 97%. The NITER temperature after 1 year was 337°C. During this period, the total yield of acrolein and acrylic acid was 92%.

One year after this stationary operation, the reactor was opened and 84 reaction tubes in total were removed from the regions near the center of the reactor shell, near the periphery and intermediate zone inside the shell at an almost same radial angle, and examined macroscopically, and no abnormality was observed in each removed catalyst.

### [Comparative Example 1]

The procedure similar to that in Example 1 including the stationary operation was conducted except for setting the propylene supply amount at 1107 Nm³/hr (corresponding to 100% of the maximum supply amount) within 15 hours after the start. The NITER temperature was targeted to a temperature corresponding to the maximum supply amount ratio in Example 1.

Once the propylene supply amount exceeded 900 Nm³/hr, the temperature of the catalyst layers could not be kept at a constant value. Since the NITER temperature became impossible to be kept at a prescribed temperature, it was set at a temperature lower by 1 to 2°C. After the propylene supply amount became 1107 Nm³/hr and the temperature of the catalyst layers became stable, the NITER temperature was set at 335°C to terminate the start-up operation.

The % propylene conversion in the stationary state was not higher than 96.5%, and the total yield of acrolein and acrylic acid was 89%.

Since the % propylene conversion was low during the stationary operation, the operation was discontinued after 1 month, and the reactor was opened and the reaction tubes were removed and examined in the manner similar to that in Example 1 described above. As a result of the examination, a part of the catalyst removed from the reaction tubes near the center of the reactor and near the circumference of the reactor exhibited a deactivation which was observed macroscopically (the condition similar to the color and the shape (shrinkage) shown empirically by a deactivated catalyst).

While the invention has been described in detail with reference to specific embodiments thereof, it will be apparent to one skilled in the art that various changes and modifications can be made therein without departing from the spirit and the scope thereof.

This application is based on the Japanese patent application filed on May 26, 2004 (Patent Application No. 2004-155840), the entire contents thereof being hereby incorporated by reference.

### <Industrial Applicability>

According to a production method of the invention, (meth) acrylic acid or (meth) acrolein can be produced at a higher yield and more stably even when supplying the raw material in an amount close to the maximum supply amount acceptable by a reactor. The resultant acrylic acid or acrylates can be used in a highly absorptive resin, coagulant, pressure-sensitive adhesive, paint, adhesive, fiber modifier and the like.

## Claims

1. A method for producing (meth)acrylic acid or (meth)acrolein by conducting a gas phase catalytic oxidation reaction with an oxygen-containing gas using as a raw material at least one substance to be oxidized selected from propylene, propane, isobutylene and (meth)acrolein using a multi-tubular reactor,
wherein, at the time of a start-up of the reaction, for a period of at least 20 hours or more after the supply amount of the raw material to the reactor per unit time reached 30% or more of the acceptable maximum supply amount of the raw material per unit time, the supply amount of the raw material per unit time is kept at 30% or more and less than 80% of the acceptable maximum supply amounts.
